(19) Europäisches Patentamt
European Patent Office
Office européen des brevets

(11) Veröffentlichungsnummer: **0 144 832**
**A2**

# (12) EUROPÄISCHE PATENTANMELDUNG

(21) Anmeldenummer: **84113800.1**

(22) Anmeldetag: **15.11.84**

(51) Int. Cl.[4]: **C 12 P 7/62**
C 12 P 7/42, C 12 P 41/00

(30) Priorität: **06.12.83 CH 6510/83**

(43) Veröffentlichungstag der Anmeldung:
**19.06.85 Patentblatt 85/25**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Leuenberger, Hans Georg Wilhelm, Dr.**
**Rebenstrasse 23**
**CH-4112 Bättwil(CH)**

(72) Erfinder: **Matzinger, Peter Karl, Dr.**
**Buchenstrasse 2**
**CH-4118 Rodersdorf(CH)**

(72) Erfinder: **Seebach, Dieter, Prof. Dr.**
**Orellistrasse 3**
**CH-8044 Zürich(CH)**

(72) Erfinder: **Züger, Max F. Dr.**
**Bergacker 44**
**CH-8046 Zürich(CH)**

(74) Vertreter: **Zimmermann, Hans, Dr. et al,**
**Grenzacherstrasse 124 Postfach 3255**
**CH-4002 Basel(CH)**

## (54) Verfahren zur Herstellung optisch aktiver, alpha-substituierter 3-Hydroxypropionsäurederivate.

(57) Herstellung von optisch atkiven Verbindungen der Formel

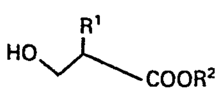

I

worin $R^1$ Alkyl, Phenyl oder Benzyl und $R^2$ Wasserstoff oder einen üblichen Esterrest bedeuten,
durch fermentative Reduktion von Vernindungen der Formel

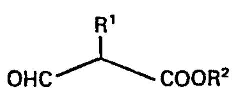

II

worin $R^1$ und $R^2$ die obigen Bedeutungen haben.

Die erhaltenen Verbindungen sind wertvolle Zwischen-producte in organischen Synthesen.

EP 0 144 832 A2

0144832

RAN 4120/028

F.HOFFMANN-LA ROCHE & CO. Aktiengesellschaft, Basel (Schweiz)

# Verfahren zur Herstellung optisch aktiver, α-substituierter 3-Hydroxypropionsäurederivate

Die Erfindung betrifft ein mikrobiologisches Verfahren zur Herstellung von optisch aktiven, α-substituierten Derivaten der 3-Hydroxypropionsäure. Die erfindungsgemäss erhältlichen Verbindungen sind wertvolle Zwischenprodukte in organischen Synthesen und können beispielsweise zur Herstellung von α-Tocopherol, Musconen, Pharmazeutika, Insektiziden, Fruchtfall induzierenden Mitteln und dergleichen verwendet werden.

Das erfindungsgemässe Verfahren ist dadurch gekennzeichnet, dass man zur Herstellung von optisch aktiven 3-Hydroxypropionsäurederivaten der allgemeinen Formel

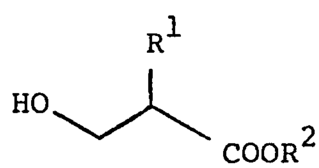

I

worin $R^1$ Alkyl, Phenyl oder Benzyl bedeutet und $R^2$ Wasserstoff oder einen üblichen Esterrest bezeichnet,

Zim/2.10.84

eine Verbindung der allgemeinen Formel

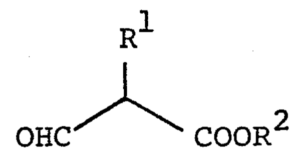

II

worin $R^1$ und $R^2$ die obigen Bedeutungen haben, fermentativ reduziert.

Der Ausdruck "Alkyl" für $R^1$ umfasst vorzugsweise geradkettige oder verzweigte Alkylgruppen mit 1 bis 6 Kohlenstoffatomen, wie Methyl, Aethyl, Propyl, Isopropyl, Butyl und dergleichen. Besonders bevorzugte Reste $R^1$ sind Phenyl und insbesondere Methyl. Die Formel I entspricht in den beiden letzteren Fällen der Tropasäure und der 3-Hydroxyisobuttersäure bzw. deren Estern.

Der Ausdruck "üblicher Esterrest" umfasst die in Carbonsäureestern üblicherweise vorkommenden Reste, wie Alkyl, Aryl, Aralkyl, Alkylaryl und dergleichen, beispielsweise Methyl, Aethyl, Isopropyl, t-Butyl, Phenyl, Benzyl und Tolyl. Vorzugsweise bedeutet $R^2$ in den obigen Formeln I und II Wasserstoff, $C_1$-$C_6$-Alkyl, Phenyl oder Benzyl, insbesondere Methyl , Aethyl oder t-Butyl.

Das erfindungsgemässe Verfahren eröffnet einen neuen vorteilhaften Weg zur Herstellung optisch aktiver Verbindungen der Formel I. Die beobachteten guten Ausbeuten bei Verwendung von racemischem Ausgangsmaterial der Formel II deuten darauf hin, dass die erfindungsgemässe Reduktion vollständig oder zumindest grösstenteils über die Enolform der Verbindungen der Formel II verläuft und daher die Konfiguration des Produktes der Formel I durch die Konfiguration des Ausgangsmaterials der Formel II nicht oder nur unwesentlich beeinflusst wird.

Das erfindungsgemässe Verfahren wird zweckmässig in der Weise durchgeführt, dass ein die Verbindungen der Formel I erzeugender Mikroorganismus in einem wässrigen Nährmedium in Anwesenheit einer Verbindung der Formel II gezüchtet wird, bzw. nach der Anzucht in Anwesenheit einer Verbindung der Formel II weiter inkubiert wird.

Je nach verwendetem Mikroorganismus wird dabei vorwiegend die (R)- oder (S)-Form der Verbindungen der Formel I gebildet. Geeignete, die Verbindungen der Formel I erzeugende Mikroorganismen können leicht durch Austesten von beliebigen Mikroorganismen - vorzugsweise aeroben oder fakultativ aeroben Hefen, Pilzen oder Bakterien - auf das erfindungsgemäss eingesetzte Edukt der Formel II gefunden werden. Vorzugsweise wird das erfindungsgemässe Verfahren zur Herstellung der (R)-Form der Verbindungen der Formel I verwendet.

Beispiele bevorzugt verwendbarer Mikroorganismen sind:

1. <u>Prokaryonten</u>

1.1. <u>Bakterien der Gattung:</u>

Pseudomonas
wie Pseudomonas aeruginosa (z.B. NRRL B771)
" aurofaciens (z.B. ATCC 13985)
" denitrificans (z.B. NRRL B1028)
" fluorescens (z.B. ATCC 13430)
" oleovorans (z.B. ATCC 8062)
" piscicida (z.B. ATCC 15251)
" putida (z.B. ATCC 21244)
" rubescens (z.B. ATCC 12099)
" saccharophila (z.B. ATCC 15946)
" testosteroni (z.B. ATCC 11996)

1.2. Mycelbildende Bakterien (Actinomyceten) der Gattung:

Nocardia
wie Nocardia asteroides (z.B. IMRU 833)
" brasiliensis (z.B. IMRU 536)
" opaca (z.B. CBS 26639)

Streptomyces
wie Streptomyces aureus (z.B. ATCC 9309)
" clavuligerus (z.B. NRRL 3585)
" eurocidicus (z.B. NRRL B1676)
" fradiae (z.B. NRRL B1195)
" flavochromogenans (z.B. CBS 74472)
" griseus (z.B. IMRU 3570, NRRL 15727)
" hirsutus (z.B. ETH 16281)
" hydrogenans (z.B. ATCC 19631)
" mediocidicus (z.B. ATCC 13278)
" olivaceus (z.B. NRRL B-1125)
" rimosus (z.B. ATCC 10970)
" roseochromogenus (z.B. ATCC 13400)
" sp. (ETH 19121, NRRL 15728)
" viridochromogenes (z.B. NRRL B1511)

2. Eukaryonten:

2.1. Pilze der Gattung:

Aspergillus
wie Aspergillus niger (z.B. ATCC 11394)
" ochraceus (z.B. NRRL 398)

Byssochlamis
wie Byssochlamis fulva (z.B. NRRL 3493)

Curvularia
wie Curvularia lunata (z.B. NRRL 2380)

Fusarium
wie Fusarium solani (z.B. ATCC 12823)

Geotrichum
wie Geotrichum candidum (z.B. CBS 23376)

Gibberella
wie Gibberella baccata (z.B. ATCC 11857)
" fujikuroi (z.B. ATCC 14842)

Gliocladium
wie Gliocladium roseum (z.B. NRRL 8194)

Mucor
wie Mucor circinelloides (z.B. ETH 2605)

Paecilomyces
wie Paecilomyces carneus (z.B. NRRL 11054)

Penicillium
wie Penicillium brevi-compactum (z.B. ATCC 32005)
" citrinum (z.B. ATCC 9849)
" lilacinum (z.B. ATCC 10114)
" notatum (z.B. NRRL 832)
" purpurogenum (z.B. NRRL 1059)

2.2. Hefen der Gattung:

Candida
wie Candida albicans (z.B. ATCC 18804)
" parapsilosis (z.B. CBS 1954)
" humicola (z.B. NRRL Y-1266)
" tropicalis (z.B. ATCC 9968)

Cryptococcus
wie Cryptococcus laurentii (z.B. IFO 0609)

Rhodosporidium
wie Rhodosporidium toruloides (z.B. ATCC 10657)

Rhodotorula
wie Rhodotorula rubra (z.B. IFO 0714)
" glutinis (z.B. NRRL Y-15726)

Saccharomyces
wie Saccharomyces cerevisiae (z.B. ATCC 7754)
" rouxii (z.B. CBS 710)

Schizosaccharomyces
wie Schizosaccharomyces pombe (z.B. CBS 1058)

Torulopsis
wie Torulopsis aeria
" magnoliae (z.B. NRRL Y-2024)
" rotundata (z.B. NRRL 41402)

Die obigen Angaben in Klammern beziehen sich jeweils auf entsprechende Stämme, die bei einer der folgenden Hinterlegungsstellen unter der angegebenen Nummer hinterlegt sind:

NRRL = Northern Utilization Research and Development Division of U.S.D.A., Peoria, Illinois, USA

ATCC = American Type Culture Collection, Rockville, Maryland, USA

CBS = Centraal-Bureau voor Schimmelcultures, Baarn, Holland

ETH = Eidgenössische Technische Hochschule, Zürich, Schweiz

IFO = Institute for Fermentation, 54-4 Juso Nishino-machi, Higashiyodogawaku, Osaka, Japan

IMRU = Institute of Microbiology, Rutgers-The State University, New Brunswick, N.J., USA

Vorzugsweise werden Mikroorganismen verwendet, welche das gewünschte Produkt der Formel I in einer optischen Reinheit von mindestens etwa 90% ee liefern. Bevorzugt verwendbare Mikroorganismen im erfindungsgemässen Verfahren sind mycelbildende Bakterien und Hefen, insbesondere Mikroorganismen der Gattungen Nocardia und Streptomyces bzw. Candida, Rhodotorula und Torulopsis. Besonders bevorzugt sind Mikroorganismen der Species Streptomyces roseochromogenus, Candida humicola, Streptomyces hydrogenans, Streptomyces griseus, Streptomyces sp. und Rhodotorula glutinis, insbesondere die oben angegebenen hinterlegten Stämme und Mutanten davon. Zur Sicherstellung der Durchführbarkeit wurden Stämme der drei letztgenannten Species am 30. November 1983 bei NRRL unter den Nummern 15727, 15728 und Y-15726 neu hinterlegt.

Es versteht sich, dass jeder der erfindungsgemäss eingesetzten Mikroorganismen vor der Verwendung in der erfindungsgemässen Fermentation angezüchtet werden soll. Die Anzucht erfolgt in der Regel in an sich bekannter Weise in einem wässrigen Medium unter Zuhilfenahme der üblichen Nährstoffe, d.h. in Gegenwart einer Kohlenstoffquelle (z.B. Glucose, Fructose, Saccharose, Maltose und/oder Malzextrakt), einer Stickstoffquelle (z.B. Harnstoff, Pepton, Hefeextrakt, Fleischextrakt, Aminosäuren und/oder Ammoniumsalze) und gegebenenfalls anorganischer Salze (z.B. Magnesium-, Natrium-, Kalium-, Kalzium- und/oder Ferrosalze) und anderer wachstumsfördernder Substanzen (z.B. Vitamine).

Das Anzuchtmedium mit dem darin gewachsenen Mikroorganismus kann in der Regel direkt für die erfindunsgemässe Reduktion verwendet werden. Die Fermentation ist dann nach Zugabe des Eduktes der Formel II ohne weitere Zusätze durchführbar.

Die Zusammensetzung des erfindunsgemäss verwendeten Fermentationsmediums kann aber auch wesentlich einfacher sein und z.B. lediglich aus einer Lösung des Eduktes der Formel II und dem verwendeten Mikroorganismus bestehen. Es ist jedoch vorteilhaft, dem wässrigen Medium eine assimilierbare Kohlenstoffquelle (beispielsweise in Form eines Zuckers, wie Glucose, Fructose, Saccharose, Maltose und dergleichen) als Mikroorganismennährstoff zuzusetzen, damit die Lebensfähigkeit und die damit verbundene Stoffwechselaktivität des Mikroorganismus möglichst lange erhalten bleiben. Vorzugsweise wird die Kohlenstoffquelle in einer Menge von etwa 10-100 g/l zugesetzt. Bei langen Fermentationszeiten ist es von Vorteil, die Kohlenstoffquelle in einer bevorzugten Menge von 10-100 g/l einmal oder mehrmals nachzufüttern.

Der Zusatz einer Stickstoffquelle zum Fermentationsmedium ist nicht notwendig. Gewünschtenfalls kann aber eine assimilierbare Stickstoffquelle (beispielsweise in Form von Harnstoff, Pepton, Hefeextrakt, Fleischextrakt, Aminosäuren, Ammoniumsalzen und dergleichen) zugesetzt werden, vorzugsweise in einer Menge von etwa 1-50 g/l. Das Fermentationsmedium kann ferner auch anorganische Salze, wie Magnesium-, Natrium-, Kalium-, Kalzium- und/oder Ferrosalze, und andere wachstumsfördernde Substanzen, wie Vitamine und dergleichen, enthalten.

Der pH-Wert des Fermentationsmediums soll vorzugsweise innerhalb des Bereiches von 2 bis 10, insbesondere 3 bis 8 liegen, ein Bereich, der zumeist ohne besondere Zusätze erreichbar ist. Gewünschtenfalls kann der pH-Wert durch

Verwendung von Puffern, Säuren oder Basen, z.B. Phosphat-, Phthalat- oder Trispuffern [Tris-(Hydroxymethyl)-aminomethan], Salzsäure, Natronlauge und dergleichen, eingestellt werden. Die Temperatur kann in weiten Grenzen schwanken, z.B. zwischen 10°C und 40°C, vorzugsweise zwischen 20°C bis 35°C.

Um optimale Ausbeuten zu erhalten, ist es vorteilhaft, das Edukt der Formel II in der Gärbrühe in einer Konzentration von etwa 0,05-5,0 Gew.-%, insbesondere etwa 0,1-0,5 Gew.-%, einzusetzen. Nach erfolgter Reaktion kann erneut Edukt in einer bevorzugten Konzentration von etwa 0,1-0,5 Gew.-% zugesetzt werden. Dieses Verfahren lässt sich bis zur Inaktivierung der Mikroorganismen wiederholen. Das Substrat kann auch mit Hilfe einer Pumpe kontinuierlich zugefügt werden.

Die nützliche Fermentationszeit ist von den verwendeten Mikroorganismen abhängig. Sie liegt bei einmaliger Eduktzugabe im allgemeinen zwischen etwa 2 und 100 Stunden, insbesondere zwischen etwa 4 und 24 Stunden. Häufig genügen aber auch kürzere Fermentationszeiten. Bei mehrmaliger oder kontinuierlicher Eduktzugabe kann sich die Fermentationszeit entsprechend verlängern.

Die Fermentation wird vorzugsweise aerob durchgeführt, z.B. unter Rühren, Schütteln unter Luftzutritt oder mittels einer Belüftungsvorrichtung. Zur Schaumbekämpfung können die üblichen Antischaummittel, wie Siliconöle, Polyalkylenglykol-Derivate, Sojabohnenöl und dergleichen, zugesetzt werden. Vorzugsweise verwendet man einen Mikroorganismus, der sich in nicht wachsender (stationärer) Phase befindet. Die Wahl eines stationären Mikroorganismus hat den Vorteil, dass die Biotransformation nicht unter sterilen Bedingungen ausgeführt werden muss, sofern ein Nährmedium verwendet wird, das keine wesentliche Vermehrung von Mikroorganismen zulässt, beispielsweise ein Nährmedium ohne Stickstoffquelle.

Nach Beendigung der Reaktion kann das Fermentationsprodukt in üblicher Weise aus der Gärbrühe isoliert werden. Vorzugsweise kommt eine Extraktion mit einem nicht wasserlöslichen organischen Lösungsmittel in Betracht, beispielsweise mit einem aliphatischen oder cycloaliphatischen, gegebenenfalls chlorierten Kohlenwasserstoff, einem aliphatischen Ester oder einem aliphatischen Aether, wie Hexan, Cyclohexan, Methylenchlorid, Chloroform, Tetrachlorkohlenstoff, Aethylacetat, Butylacetat, Amylacetat, Diäthyläther, Diisopropyläther und dergleichen. Ein bevorzugtes Lösungsmittel ist Aethylacetat.

Das erhaltene Rohprodukt kann gewünschtenfalls in üblicher Weise, z.B. durch Destillation, Kristallisation, chromatographische Methoden etc., gereinigt werden. In vielen Fällen ist es von Vorteil, wenn das Rohprodukt vorher in ein geeignetes Derivat übergeführt wird, beispielsweise durch Veresterung der Hydroxygruppe mit 3,5-Dinitrobenzoylchlorid, einem Chlorid einer optisch aktiven Carbonsäure, wie α-Methoxy-α-trifluormethyl-phenylacetylchlorid, und dergleichen, oder durch Carbamat-Bildung, z.B. mit optisch aktivem α-Naphthyl-äthylisocyanat.

Die Ausgangsmaterialien der Formel II sind bekannte oder Analoge bekannter Verbindungen. Sie können beispielsweise nach bekannten Formylierungsreaktionen (z.B. mit Lithiumdiisopropylamid und Ameisensäureäthylester) ausgehend von den entsprechenden Carbonsäuren bzw. deren Estern erhalten werden.

Da die Verbindungen der Formel I eine "pseudosymmetrisch" chirale Struktur [Helv.Chim.Acta 60, 925 (1977)] besitzen, sind ausgehend von einem Antipoden der Verbindungen der Formel I durch rein chemische Schritte jeweils beide antipodalen Formen weiterer Schlüsselverbindungen für organische Synthesen zugänglich. Derartige Umsetzungen und weitere Verwendungsmöglichkeiten für die erfindungsgemäss hergestellten Verbindungen sind beispielsweise in Helv.Chim.Acta 60, 925 (1977) und 64,

1158 (1981); J.Org.Chem. 41, 3505 (1976); Tetrahdedron Lett. 22, 3925 (1981) und 31, 2745 (1978); Tetrahedron 37, 3873 (1981); J.Amer.Chem.Soc. 101, 6789 (1979), 102, 2117-21 (1980), 104, 4496 (1982) und 104, 5528 (1982) und in der Britischen Patentpublikation Nr. 1229259 ausführlich beschrieben. Durch Kettenverlängerung mittels bekannter C-C-Verknüpfungsmethoden (z.B. Wittig- oder Grignard-Reaktionen) sind ferner Insektizide, wie z.B. die in Chemtech 1976, 358 beschriebenen, zugänglich.

Das erfindungsgemässe Verfahren wird durch die folgenden Beispiele weiter veranschaulicht. Die Edukte wurden jeweils in racemischer Form eingesetzt. Die erhaltenen Produkte wurden in den Beispielen 1-7 und 10-12 jeweils mit α-Methoxy-α-trifluormethyl-phenylacetyl-chlorid [J.Org.Chem. 34, 2543 (1969)] verestert bzw. in den Beispielen 8, 9 und 14 mit (S)-(+)-α-Naphthyläthylisocyanat [z.B. J.Org.Chem. 39, 3904 (1974)] zum Carbamat umgesetzt und dann die optische Reinheit der Produkte anhand der NMR-Spektren und Gaschromatogramme dieser Ester bzw. Carbamate bestimmt.

Beispiel 1

Die Hefe Candida humicola NRRL Y-1266 wurde auf einem Schrägagar mit 7,5 ml Medium 1 bestehend aus 1 Gew.-% Hefeextrakt, 1 Gew.-% Pepton, 2 Gew.-% Glucose, 1,6 Gew.-% Agar und 94,4 Gew.-% destilliertem Wasser gezüchtet. Nach 3 Tagen Inkubation bei 30°C wurde der Ansatz mit 9 ml 0,9%-iger (Gew./Vol.) Kochsalzlösung versetzt und die Zellen suspendiert. Anschliessend wurde ein Schüttelkolben enthaltend 100 ml Medium 1 ohne Zusatz von Agar (d.h. ein Medium bestehend aus 1 Gew.-% Hefeextrakt, 1 Gew.-% Pepton, 2 Gew.-% Glucose und 96 Gew.-% destilliertem Wasser) mit 1 ml der erhaltenen Suspension beimpft. Nach einer Inkubation von 3 Tagen unter Schütteln bei 30°C wurden die Zellen abzentrifugiert und in 100 ml 5%-iger (Gew./Vol.) Glucoselösung suspendiert. Die erhaltene Suspension wurde mit 0,1 g α-Formylpropionsäureäthylester versetzt und unter Schütteln bei 30°C weiterinkubiert. Nach 5 Stunden wurde der Ansatz mit Aethylacetat extrahiert, wobei (R)-(-)-3-Hydroxy-isobuttersäureäthylester in einer optischen Reinheit von 95,2% ee erhalten wurde.

Beispiel 2

Die Hefe Rhodotorula glutinis NRRL Y-15726 wurde auf einem Schrägagar mit 7,5 ml Medium 1 bestehend aus 1 Gew.-% Hefeextrakt, 1 Gew.-% Pepton, 2 Gew.-% Glucose, 1,6 Gew.-% Agar und 94,4 Gew.-% destilliertem Wasser gezüchtet. Nach 3 Tagen Inkubation bei 30°C wurde der Ansatz mit 9 ml 0,9%-iger (Gew./Vol.) Kochsalzlösung versetzt und die Zellen suspendiert. Anschliessend wurde ein Schüttelkolben enthaltend 100 ml Medium 1 ohne Zusatz von Agar (d.h. ein Medium bestehend aus 1 Gew.-% Hefeextrakt, 1 Gew.-% Pepton, 2 Gew.-% Glucose und 96 Gew.-% destilliertem Wasser) mit 1 ml der erhaltenen Suspension beimpft. Nach einer Inkubation von 24 Stunden unter Schütteln bei 30°C wurde die Vorkultur mit 0,1 g α-Formylpropionsäureäthylester ver-

setzt und unter Schütteln bei 30°C weiterinkubiert. Nach 24 Stunden wurde der Ansatz mit Aethylacetat extrahiert, wobei (R)-(-)-3-Hydroxy-isobuttersäureäthylester in einer optischen Reinheit von 96,8% ee erhalten wurde.

## Beispiel 3

Streptomyces roseochromogenus ATCC 13400 wurde auf einem Schrägagar mit 7,5 ml Medium 2 gezüchtet, welches aus 0,4 Gew.-% Hefeextrakt, 1 Gew.-% Malzextrakt, 0,4 Gew.-% Saccharose, 1,5 Gew.-% Agar und 96,7 Gew.-% Leitungswasser bestand und mit Natronlauge auf einen pH-Wert von 7,3 eingestellt worden war. Nach 6 Tagen Inkubation bei 30°C wurde der Ansatz mit 9 ml 0,9%-iger (Gew./Vol.) Kochsalzlösung versetzt. Anschliessend wurden 100 ml Medium 3 bestehend aus 1 Gew.-% Cornsteep, 1 Gew.-% Sojamehl, 1 Gew.-% Glucose und 97 Gew.-% destilliertem Wasser mit 1 ml der erhaltenen Sporensuspension angeimpft. Nach einer Inkubation von 3 Tagen unter Schütteln bei 30°C wurde 0,1 g α-Formylpropionsäureäthylester direkt zur Vorkultur gegeben und unter Schütteln bei 30°C weiterinkubiert. Nach 4 Stunden Reaktionszeit wurde der Ansatz mit Aethylacetat extrahiert, wobei (R)-(-)-3-Hydroxy-isobuttersäureäthylester in einer optischen Reinheit von 89,8% ee erhalten wurde.

## Beispiel 4

Streptomyces griseus NRRL 15727 wurde auf einem Schrägagar mit 7,5 ml Medium 2 gezüchtet, welches aus 0,4 Gew.-% Hefeextrakt, 1 Gew.-% Malzextrakt, 0,4 Gew.-% Saccharose, 1,5 Gew.-% Agar und 96,7 Gew.-% Leitungswasser bestand und mit Natronlauge auf einen pH-Wert von 7,3 eingestellt worden war. Nach 6 Tagen Inkubation bei 30°C wurde der Ansatz mit 9 ml 0,9%-iger (Gew./Vol.) Kochsalzlösung versetzt. Anschliessend wurden 100 ml Medium 4, welches aus

2 Gew.-% Glucose, 0,5 Gew.-% Pepton, 0,5 Gew.-% Fleischextrakt, 0,3 Gew.-% Hefeextrakt, 0,3 Gew.-% Kalziumcarbonat und 96,4 Gew.-% destilliertem Wasser bestand und mit Natronlauge auf einen pH-Wert von 7,0 eingestellt worden war, mit 1 ml der erhaltenen Sporensuspension angeimpft. Nach einer Inkubation von 3 Tagen unter Schütteln bei 30°C wurde 0,1 g α-Formylbuttersäureäthylester direkt zur Vorkultur gegeben und unter Schütteln bei 30°C weiterinkubiert. Nach 24 Stunden Reaktionszeit wurde der Ansatz mit Aethylacetat extrahiert, wobei (R)-(-)-2-(Hydroxymethyl)buttersäureäthylester in einer optischen Reinheit von 88,4% ee erhalten wurde.

## Beispiel 5

Streptomyces griseus NRRL 15727 wurde wie in Beispiel 4 mit Medium 2 und 4 angezüchtet. Nach einer Inkubation von 3 Tagen unter Schütteln bei 30°C wurde schliesslich 0,1 g α-Formylisovaleriansäureäthylester direkt zur Vorkultur (Medium 4) gegeben und unter Schütteln bei 30°C weiterinkubiert. Nach 24 Stunden Reaktionszeit wurde der Ansatz mit Aethylacetat extrahiert, wobei (R)-(-)-2-(Hydroxymethyl)isovaleriansäureäthylester in einer optischen Reinheit von 93% ee erhalten wurde.

## Beispiel 6

Streptomyces griseus NRRL 15727 wurde wie in Beispiel 4 mit Medium 2 und 4 angezüchtet. Nach einer Inkubation von 3 Tagen unter Schütteln bei 30°C wurde schliesslich 0,1 g α-Formyl-α-phenylessigsäureäthylester direkt zur Vorkultur (Medium 4) gegeben und unter Schütteln bei 30°C weiterinkubiert. Nach 4 Stunden Reaktionszeit wurde der Ansatz mit Aethylacetat extrahiert, wobei R-(+)-Tropasäureäthylester [R-(+)-3-Hydroxy-2-phenylpropionsäureäthylester] in einer optischen Reinheit von 95,6% ee erhalten wurde.

## Beispiel 7

Streptomyces sp. NRRL 15728 wurde in analoger Weise zu Beispiel 4 mit Medium 2 und 4 angezüchtet. Nach einer Inkubation von 3 Tagen unter Schütteln bei 30°C wurde schliesslich 0,1 g α-Formylpropionsäureäthylester direkt zur Vorkultur (Medium 4) gegeben und unter Schütteln bei 30°C weiterinkubiert. Nach 24 Stunden Reaktionszeit wurde der Ansatz mit Aethylacetat extrahiert, wobei (R)-(-)-3-Hydroxyisobuttersäureäthylester in einer optischen Reinheit von 93,4% ee erhalten wurde.

## Beispiel 8

Die Hefe Candida humicola NRRL Y-1266 wurde auf einem Schrägagar mit 7,5 ml Medium 1 bestehend aus 1 Gew.-% Hefeextrakt, 1 Gew.-% Pepton, 2 Gew.-% Glucose, 1,6 Gew.-% Agar und 94,4 Gew.-% destilliertem Wasser gezüchtet. Nach 3 Tagen Inkubation bei 30°C wurde der Ansatz mit 9 ml 0,9%-iger (Gew./Vol.) Kochsalzlösung versetzt und die Zellen suspendiert. Anschliessend wurde ein Schüttelkolben enthaltend 100 ml Medium 1 ohne Zusatz von Agar (d.h. ein Medium bestehend aus 1 Gew.-% Hefeextrakt, 1 Gew.-% Pepton, 2 Gew.-% Glucose und 96 Gew.-% destilliertem Wasser) mit 1 ml der erhaltenen Suspension beimpft. Nach einer Inkubation von 24 Stunden unter Schütteln bei 30°C wurde die Vorkultur mit 0,1 g α-Formyl-β-phenylpropionsäureäthylester versetzt und unter Schütteln bei 30°C weiterinkubiert. Nach 1 Stunde wurde der Ansatz mit Aethylacetat extrahiert, wobei (R)-(+)-2-Benzyl-3-hydroxypropionsäureäthylester in einer optischen Reinheit von 73,6% ee isoliert wurde.

Beispiel 9

Penicillium notatum NRRL 832 wurde auf einem Schrägagar mit 7,5 ml Medium 5 bestehend aus 2 Gew.-% Sucrose, 1,5 Gew.-% Agar und 96,5 Gew.-% Bierwürze (spez. Gew. 1,05-1,035) mit einem pH von 6,5 gezüchtet. Nach 6 Tagen Inkubation bei 30°C wurde der Ansatz mit 9 ml 0,9%-iger (Gew./Vol.) Kochsalzlösung versetzt und die Zellen suspendiert. Anschliessend wurde ein Schüttelkolben enthaltend 100 ml Medium 6, welches aus 1 Gew.-% Cornsteep, 1 Gew.-% Sojamehl, 1 Gew.-% Glucose und 97 Gew.-% destilliertem Wasser bestand, mit 1 ml der erhaltenen Suspension beimpft. Nach einer Inkubation von 3 Tagen unter Schütteln bei 30°C wurde 0,1 g α-Formyl-β-phenylpropionsäureäthylester direkt zur Vorkultur gegeben und unter Schütteln bei 30°C weiterinkubiert. Nach 1 Stunde Reaktionszeit wurde der Ansatz mit Aethylacetat extrahiert, wobei (R)-(+)-2-Benzyl-3-hydroxypropionsäureäthylester in einer optischen Reinheit von 88,5% ee erhalten wurde.

Beispiel 10

Paecilomyces carneus NRRL 11054 wurde wie in Beispiel 9 mit Medium 5 und 6 angezüchtet. Nach einer Inkubation von 3 Tagen unter Schütteln bei 30°C wurde 0,1 g α-Formylbuttersäureäthylester direkt zur Vorkultur gegeben und unter Schütteln bei 30°C weiterinkubiert. Nach 1 Stunde Reaktionszeit wurde der Ansatz mit Aethylacetat extrahiert, wobei (R)-(-)-2-(Hydroxymethyl)buttersäureäthylester in einer optischen Reinheit von 86,5% ee erhalten wurde.

Beispiel 11

Streptomyces hydrogenans ATCC 19631 wurde wie in Beispiel 4 mit Medium 2 und 4 angezüchtet. Nach einer Inkubation von 3 Tagen unter Schütteln bei 30°C wurde schliesslich 0,1 g α-Formylisovaleriansäureäthylester

direkt zur Vorkultur (Medium 4) gegeben und unter Schütteln bei 30°C weiterinkubiert. Nach 24 Stunden Reaktionszeit wurde der Ansatz mit Aethylacetat extrahiert, wobei (R)-(-)-2-(Hydroxymethyl)isovaleriansäureäthylester in einer optischen Reinheit von 96,5% ee erhalten wurde.

## Beispiel 12

Streptomyces hydrogenans ATCC 19631 wurde wie in Beispiel 4 mit Medium 2 und 4 angezüchtet. Nach einer Inkubation von 3 Tagen unter Schütteln bei 30°C wurde schliesslich 0,1 g α-Formyl-α-phenylessigsäureäthylester direkt zur Vorkultur (Medium 4) gegeben und unter Schütteln bei 30°C weiterinkubiert. Nach 24 Stunden Reaktionszeit wurde der Ansatz mit Aethylacetat extrahiert, wobei R-(+)-Tropasäureäthylester [R-(+)-3-Hydroxy-2-phenylpropionsäureäthylester] in einer optischen Reinheit von 95,5% ee erhalten wurde.

## Beispiel 13

Die Hefe Candida albicans ATCC 18804 wurde in analoger Weise zu Beispiel 8 mit Medium 1 angezüchtet. Nach einer Inkubation von 1 Tag unter Schütteln bei 30°C wurde 0,1 g α-Formyl-buttersäureäthylester direkt zur Vorkultur gegeben. Nach 1 Stunde Reaktionszeit wurde der Ansatz mit Aethylacetat extrahiert, wobei (S)-(+)-2-(Hydroxymethyl)buttersäureäthylester in einer optischen Reinheit von 76% ee erhalten wurde.

## Beispiel 14

Streptomyces hydrogenans ATCC 19631 wurde wie in Beispiel 4 mit Medium 2 und 4 angezüchtet. Nach einer Inkubation von 3 Tagen unter Schütteln bei 30°C wurde schliesslich 0,1 g α-Formyl-β-phenylpropionsäureäthylester

direkt zur Vorkultur gegeben. Nach 24 Stunden Reaktionszeit wurde der Ansatz mit Aethylacetat extrahiert, wobei (R)-(+)-2-Benzyl-3-hydroxypropionsäureäthylester in einer optischen Reinheit von 99% ee erhalten wurde.

## Patentansprüche

1. Verfahren zur Herstellung von optisch aktiven 3-Hydroxypropionsäurederivaten der allgemeinen Formel

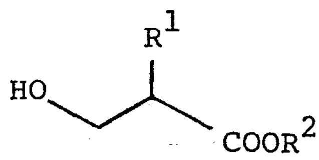

I

worin $R^1$ Alkyl, Phenyl oder Benzyl bedeutet und $R^2$ Wasserstoff oder einen üblichen Esterrest bezeichnet, dadurch gekennzeichnet, dass man eine Verbindung der allgemeinen Formel

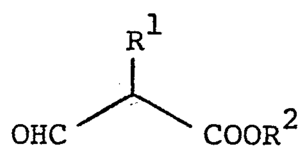

II

worin $R^1$ und $R^2$ die obigen Bedeutungen haben, fermentativ reduziert.

2. Verfahren nach Anspruch 1, dadurch gekennzeichnet, dass ein die Verbindung der Formel I erzeugender Mikroorganismus in einem wässrigen Nährmedium in Anwesenheit einer Verbindung der Formel II gezüchtet wird oder nach der Anzucht in Anwesenheit einer Verbindung der Formel II weiter inkubiert wird.

3. Verfahren nach Anspruch 1 oder 2, dadurch gekennzeichnet, dass man die Reaktion mit einem Mikroorganismus der Gattung Nocardia, Streptomyces, Candida, Rhodotorula oder Torulopsis durchführt.

4. Verfahren nach einem der Ansprüche 1 bis 3, dadurch gekennzeichnet, dass man die Reaktion mit einem Mikroorganismus der Species Nocardia asteroides, Nocardia opaca, Streptomyces flavochromogenans, Streptomyces griseus,

Streptomyces hirsutus, Streptomyces hydrogenans, Streptomyces mediocidicus, Streptomyces rimosus, Streptomyces roseochromogenus, Streptomyces sp., Aspergillus ochraceus, Paecilomyces carneus, Penicillium notatum, Candida humicola, Rhodotorula ruba, Rhodotorula gluticis, Saccharomyces cerevisiae, Torulopsis aeria, Torulopsis magnoliae oder Torulopsis rotundata durchführt.

5. Verfahren nach einem der Ansprüche 1 bis 4, dadurch gekennzeichnet, dass man die Reaktion mit Streptomyces griseus NRRL 15727, Streptomyces roseochromogenus ATCC 13400, Streptomyces sp. NRRL 15728, Streptomyces hydrogenans ATCC 19631, Candida humicola NRRL Y-1266, Rhodotorula glutinis NRRL Y-15726 oder einer Mutante davon durchführt.

6. Verfahren nach einem der Ansprüche 1 bis 5, dadurch gekennzeichnet, dass man die Reaktion mit einer Verbindung der Formel II durchführt, worin $R^2$ Wasserstoff, $C_1$-$C_6$-Alkyl, Phenyl oder Benzyl bedeutet.

7. Verfahren nach Anspruch 6, dadurch gekennzeichnet, dass man die Reaktion mit einer Verbindung der Formel II durchführt, worin $R^2$ Methyl, Aethyl oder t-Butyl bedeutet.

8. Verfahren nach einem der Ansprüche 1 bis 7, dadurch gekennzeichnet, dass man die Reaktion mit einer Verbindung der Formel II durchführt, worin $R^1$ Methyl oder Phenyl bedeutet.

9. Verfahren nach einem der Ansprüche 1 bis 8, dadurch gekennzeichnet, dass man die Reaktion bei einem pH-Wert von 3 bis 8 durchführt.

10. Verfahren nach einem der Ansprüche 1 bis 9, dadurch gekennzeichnet, dass man die Reaktion bei einer Temperatur von 20°C bis 35°C durchführt.

***